# FASCICULE DE BREVET EUROPEEN

(11) **EP 1 328 167 B1**
(45) Date de publication et mention de la délivrance du brevet: **04.10.2006**
(21) Numéro de dépôt: 01978716.7
(22) Date de dépôt: 22.10.2001
(51) Int. Cl.: A23L 3/32, A61L 2/03, C02F 1/48

(54) **PROCEDE ET DISPOSITIF DE STERILISATION**
VERFAHREN UND VORRICHTUNG ZUR STERILISATION
METHOD AND DEVICE FOR STERILISING A LIQUID

(30) Priorité: 27.10.2000 EP 00810998
(43) Date de publication de la demande: 23.07.2003
(73) Titulaire: Apit Corp. SA, 1950 Sion (CH)
(72) Inventeur: KOULIK, Pavel, F-67113 Blaesheim (FR); KRAPIVINA, Svetlana, F-67400 Illdirch-Graffenstaden (FR); SAITCHENKO, Anatolii, F-67400 Illkirch-Graffenstaden (FR)
(74) Mandataire: Reuteler, Raymond Werner
(86) Numéro de dépôt international: PCT/IB2001/001979
(87) Numéro de publication internationale: WO 2002/034075

(56) Documents cités:
- US-A- 4 695 472
- US-A- 5 415 882
- US-A- 6 050 178
- MERTENS B ET AL: "DEVELOPMENTS OF NONTHERMAL PROCESSES FOR FOOD PRESERVATION" FOOD TECHNOLOGY, INSTITUTE OF FOOD TECHNOLOGISTS. CHICAGO, US, vol. 46, no. 5, 1 mai 1992 (1992-05-01), pages 124,126-133, XP000278073 ISSN: 0015-6639

## Description

L'invention concerne un procédé de stérilisation d'un liquide ou d'un objet solide en contact avec un liquide et d'un dispositif pour la mise en oeuvre de ce procédé.

L'invention concerne notamment un procédé de stérilisation d'une solution aqueuse ainsi que de surfaces en contact avec ce liquide, contaminées notamment par des levures ou moisissures.

Plusieurs méthodes de stérilisation d'un liquide ainsi que les surfaces en contact avec ce liquide sont connues. Une des méthodes consiste en l'utilisation de rayonnements pénétrants, tels que des rayons ultraviolets, des rayons X, des rayons γ et des rayons β. Les rayonnements UV sont relativement lents et ne permettent pas de traiter les zones d'ombre. Ils sont en outre susceptibles de modifier l'état de la structure des molécules dont les énergies d'excitation sont inférieures à 2eV. Les autres rayonnements ionisants sont très souvent nocifs pour le produit du fait qu'ils peuvent modifier leur propriétés physico-chimiques, détruire certaines molécules, ou créer des états excités qui peuvent nuire à la santé des consommateurs (voir Bernard D. T., Gravin A., Scott V.N., Shafer B.D., Stevenson K.E., Unverferth I.A. et Chandarana D.I. "Validation of Aseptic Processing and Packaging". Food Technology, 1990, 12 p. 119-122).

D'autres procédés de stérilisation bien connus utilisent des produits chimiques pour tuer les micro-organismes. L'utilisation de produits chimiques de désinfection ou de stérilisation est sévèrement contrôlée en raison des effets néfastes de certains produits sur l'environnement et la santé des consommateurs. Dans l'industrie alimentaire par exemple, la tendance est à la réduction, voire même à l'élimination des produits préservants.

Les procédés de stérilisation thermiques, tels que la pasteurisation, sont très répandus, mais ont le désavantage de dégrader les produits, ce qui peut conduire, en ce qui concerne les produits alimentaires, à une modification de leurs propriétés sensorielles et nutritives et à la réduction des vitamines telle que la vitamine C.

La pasteurisation s'effectue normalement à des températures au-dessus de 75° C et est très souvent maintenue au-dessus de 90° C pendant plus de 60 secondes.

Le procédé de stérilisation décrit dans la publication WO 97/19707 consiste à générer des composants bactéricides, à savoir l'hypochlorite de sodium, de manière électrochimique. Ce procédé altère également les propriétés du liquide à stériliser.

Pour éviter les inconvénients des procédés précités, on a penser à développer des procédés mécaniques qui sont destinés à détruire les micro-organismes sans dégrader les propriétés physico-chimiques des liquides. Ces procédés se basent sur la création d'une différence de pression, par exemple des ultrasons, des ondes de choc ou des très hautes pressions, entre l'intérieur des microorganismes et l'extérieur pour faire éclater leurs membranes extérieures. Ces procédés sont très complexes et chers.

Il y aussi des procédés basés sur la création de champs électriques et électromagnétiques qui sont encore au stade de développement et qui ont été discutés par exemple dans les documents:
- Le Tinier Y. "Stabilisation microbiologique des aliments par champs électriques pulsés". Cours international microbiologie et maîtrise de la sécurité des aliments (4-15 mai 1998) Institut Pasteur de Lille, 1998; et
- Sitzmann W., Munch E.W. "Elektrische Hochspannungsimpulse zur Abtötung von Mikroorganismen in pumpfâhigen Nahrungsmitteln". Die Molkerei-Zeitung, 42. Jahrgang, V 48, 1998.

Les micro-organismes sont tués par l'électroporation irréversible de la membrane qui les enveloppe par l'effet de hauts champs électriques pulsés (High Density Electrical Fields - HDEF). Ces procédés, qui sont proposés principalement pour la stérilisation de boissons, nécessitent des champs électriques d'un ordre de grandeur de 10⁵ à 10⁶ V/cm agissant par une ou plusieurs impulsions dont la durée est de l'ordre de 10⁻⁵ à 10⁻⁶ sec. Le volume de liquide qui est traité est très petit, de l'ordre de quelques mm³, le liquide devant s'écouler entre une pointe d'une première électrode et d'une deuxième électrode, l'espace entre les électrodes étant de moins d'un millimètre à quelques millimètres. Outre le fait qu'un tel procédé est relativement coûteux, le potentiel électrique très élevé peut modifier les propriétés physico-chimiques du liquide par la dégradation de certaines molécules.

Tous les procédés précités, à part les procédés utilisant des rayonnements pénétrants et le procédé mécanique utilisant des ultrasons, ne peuvent pas être effectués une fois que le liquide est dans un récipient hermétiquement fermé.

Dans US-4 695 472 un procédé de sterilisation d'aliments liquides par des impulsions électriques sequentielles d'au moins 5 kV/cm jusqu'à 25 kV/cm est décrit.

Au vu des inconvénients des procédés conventionnels, un but de l'invention est de réaliser un procédé de stérilisation de liquide ou d'un objet solide plongé dans, ou en contact avec un liquide, qui n'altère pas ou peu les propriétés physico-chimiques du liquide ou du solide. Il est avantageux de réaliser un procédé de stérilisation qui permet de traiter des grands volumes de liquide de façon économique.

Il est très avantageux de pouvoir simultanément stériliser le liquide ainsi qu'un récipient hermétiquement fermé contenant ce liquide, ou d'autres surfaces en contact avec le liquide.

Il est aussi avantageux de pouvoir stériliser le liquide contenu dans un récipient conventionnel utilisé dans l'industrie alimentaire, tel qu'un récipient PET.

Il est en outre avantageux de pouvoir stériliser un liquide sans altération des qualités nutritives, et notamment des vitamines naturelles.

Un autre but de l'invention est de réaliser un dispositif pour la mise en oeuvre d'un procédé de stérilisation qui ne dégrade pas ou que peu les propriétés physico-chimiques d'un liquide ou d'un solide en contact avec un liquide. Il est en outre avantageux de réaliser un dispositif qui permet de stériliser un grand volume de liquide de façon économe.

Des buts de l'invention sont réalisés par un procédé de stérilisation d'un liquide ou d'objets solides en contact avec un liquide selon la revendication 1, et un dispositif selon la revendication 17 pour la mise en oeuvre de ce procédé.

Dans la présente invention, un procédé de stérilisation d'un liquide ou d'objets solides en contact avec un liquide comporte l'étape ou les étapes d'échauffement du liquide et d'application d'un champ électrique de l'ordre de grandeur d'environ 10² V/cm ou plus.

Dans un procédé selon l'invention, on peut avantageusement ajouter des vibrations acoustiques, de préférence dans la gamme de fréquences des ultrasons, lors du traitement de stérilisation.

Un des résultats surprenants de l'invention est qu'il suffit d'appliquer un champ électrique relativement faible pour tuer les micro-organismes, si le liquide à stériliser est chauffé à une température supérieure à une température de seuil Tₛ, la température de seuil étant bien inférieure à la température nécessaire pour une stérilisation par effet thermique seul, c'est à dire par pasteurisation.

Un autre résultat surprenant de l'invention est que l'adjonction de vibrations acoustiques pendant le procédé de traitement de stérilisation renforce l'effet de destruction des micro-organismes et permet de diminuer la température à laquelle le traitement s'effectue.

Les inventeurs ont trouvé que la température de seuil Tₛ (sans application de vibrations) pour la plupart des micro-organismes se situe entre environ 60 et 75°C. Les inventeurs ont trouvé que l'adjonction de vibrations acoustiques permet d'abaisser la température de traitement d'environ 10 à 30°C.

Un autre avantage important de l'invention est que la durée du procédé pour tuer les micro-organismes est très faible. Dans certains cas, la durée peut même être de l'ordre de grandeur d'une seconde ou moins.

Les avantages d'un procédé selon l'invention sont considérables. Premièrement, un champ électrique de l'ordre de grandeur de 10² à 10³ V/cm peut être facilement produit et appliqué à un volume de liquide relativement grand, tel qu'un volume d'un litre contenu dans une bouteille cylindrique conventionnelle utilisée dans l'industrie alimentaire. Deuxièmement, la très courte durée de stérilisation réduit le temps et donc le coût de production de liquides stérilisés, notamment en ce qui concerne le traitement de grands volumes qu'ils soient contenus dans des récipients ou non, sans dégrader les propriétés physico-chimiques du liquide. Troisièmement, la stérilisation peut s'effectuer dans le cas de liquides contenus dans des récipients hermétiquement fermés, et même des récipients en matière plastique telle que du PET, qui supporte les températures allant jusqu'à environ 75° C. Quatrièmement, la stérilisation peut s'effectuer pour des liquides à haute viscosité ou contenant des particules ou des protéines en suspension, sans risque de coagulation.

Le procédé selon l'invention permet de stériliser les surfaces d'objets solides plongés ou en contact avec un liquide sans modifier les propriétés physico-chimiques des objets solides.

Des produits alimentaires liquides, tels que des boissons, peuvent donc être stérilisés après l'opération de remplissage dans une bouteille ou un autre récipient hermétiquement fermé. Les récipients et couvercles ou capuchons ne doivent pas être stérilisés au préalable puisque le procédé selon l'invention stérilise aussi les surfaces en contact avec le liquide. A cet effet, le récipient est tourné de sorte que le liquide balaie toute la surface intérieure du récipient pendant le procédé de stérilisation. Dans le cas particulier d'une stérilisation de liquide dans des bouteilles essentiellement axisymétriques, il suffit par exemple de faire tourner la bouteille autour de son axe de symétrie, la bouteille étant disposée, par exemple, de façon à ce que cet axe de symétrie soit approximativement horizontal.

Le champ électrique peut être produit par une source de courant continu, alterné ou pulsé ou par des ondes électromagnétiques, notamment des microondes. De préférence, le champ électrique appliqué au liquide est de l'ordre de grandeur d'environ 10³ V/m et la température du liquide est d'environ 62° C à 75° C maintenue pendant une durée d'au moins environ 0,3 sec.

Le liquide peut être préchauffé à une température inférieure à 62° C avant l'étape d'échauffement à la température de stérilisation et de l'application du champ électrique. Après cette étape de stérilisation, le liquide peut être rapidement refroidi, par exemple par un dispositif d'échange de chaleur qui récupère une partie de l'énergie de chaleur et l'utilise pour préchauffer le liquide en amont.

La source de chaleur pour amener la température du liquide à 62° C ou plus peut avantageusement être du même type que la source d'énergie électrique appliquant le champ électrique. Une source micro-onde peut par exemple d'une part chauffer le liquide, et d'autre part créer un champ électrique qui peut être ajusté en fonction de la longueur d'onde et de la puissance suivant les caractéristiques du liquide à stériliser, et notamment sa conductivité ainsi que les dimensions et les formes du récipient et la vitesse de déplacement du liquide dans le champ.

La création d'un champ électrique et l'échauffement peuvent aussi être effectués par induction électromagnétique telle que produit par des spires d'un conducteur alimenté en courant électrique à impulsion unipolaire ou alternatif. L'application du champ électrique peut aussi être effectuée par deux électrodes, disposées de part et d'autre d'un volume de liquide à stériliser. La différence de potentiel, continu ou alternatif, entre électrodes produit le champ électrique. La tension appliquée aux électrodes dépend entre autres de la distance séparant les électrodes et des caractéristiques diélectriques/conductrices du liquide à stériliser.

D'autres buts et aspects avantageux de l'invention ressortiront des revendications, de la description et des exemples ci-après ainsi que des dessins annexés dans lesquels
la Fig. 1 est un schéma simplifié d'un dispositif pour la mise en oeuvre d'un procédé selon l'invention;
la Fig. 2 est une vue simplifiée en perspective d'une première variante d'une partie du dispositif pour l'application d'un champ électrique à un liquide et récipient à stériliser;
la Fig. 3 est une vue d'une autre variante d'une partie du dispositif pour l'application d'un champ électrique à un liquide et récipient à stériliser;
la Fig. 4 est une vue d'une autre variante d'une partie du dispositif pour l'application d'un champ électrique à un liquide et récipient à stériliser;
la Fig. 5 est une vue d'une autre variante d'une partie du dispositif pour l'application d'un champ électrique à un liquide et récipient à stériliser;
la Fig. 6 est une autre variante d'une partie du dispositif pour l'application d'un champ électrique à un liquide à stériliser;
la Fig. 7 est un graphique montrant le domaine d'utilisation en température et en temps de stérilisation de l'invention et d'une méthode conventionnelle de pasteurisation;
la Fig. 8 est une coupe schématique d'un mélangeur pour alimenter le liquide à stériliser par un additif permettant de varier l'inertie thermique moyenne du liquide, ce mélangeur faisant partie d'un dispositif de mise en oeuvre du procédé selon l'invention;
la Fig. 9 est une coupe schématique d'une partie du dispositif pour l'application d'un champ électrique comprenant un échangeur de chaleur pour le maintien du liquide à stériliser à une température spécifiée;
la Fig. 10 est une vue en perspective en coupe d'une autre variante d'une partie du dispositif pour l'application d'un champ électrique à un liquide à stériliser;
la Fig. 11 est un schéma simplifié d'une station de stérilisation d'un dispositif pour la mise en oeuvre d'un procédé selon l'invention; et
la Fig. 12 est une vue d'un diffuseur de micro-ondes utilisé dans le dispositif de la Fig. 11.

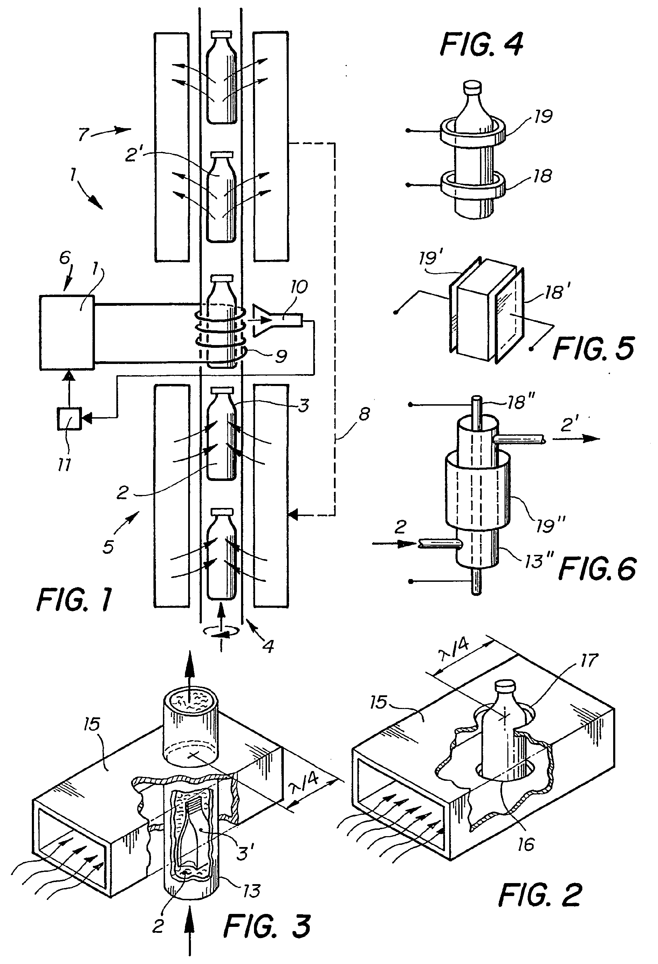

En faisant référence tout d'abord à la Fig. 1, un dispositif 1 pour la stérilisation d'un liquide 2 contenu, dans cet exemple, dans un récipient 3 hermétiquement fermé, comporte un système cinématique 4 pour le positionnement et le déplacement du produit liquide 2 et du récipient 3 aux différentes stations de traitement du dispositif, une station d'échauffement 5, une station de stérilisation 6, et une station de refroidissement 7, les stations étant disposées le long du système cinématique 4.

La station d'échauffement 5 peut comporter des éléments chauffants pour chauffer le récipient 3 par convection et/ou rayonnement infrarouge à une température, par exemple, de l'ordre de 20 à 60° C. L'élément d'échauffement peut même faire partie d'un échangeur de chaleur comprenant la station de refroidissement 7, cette dernière récupérant l'énergie de chaleur latente du liquide stérilisé 2' en aval de la station de stérilisation 6. Les stations d'échauffement et de refroidissement sont dans ce cas reliées par des conduites 8. Le principe de tels échangeurs de chaleur étant bien connu, il n'est pas nécessaire de le décrire plus en détail. Il est néanmoins à noter qu'un échangeur de chaleur permettra de récupérer jusqu'à environ 40 à 90 % de l'énergie utilisée pour l'échauffement.

Un autre avantage de ce système est que le préchauffage du liquide diminue la puissance à fournir par la station de stérilisation 6 et/ou le temps de stérilisation. Le refroidissement rapide du liquide après stérilisation permet non seulement d'économiser de l'énergie, mais également de réduire les effets de dégradation des propriétés physico-chimiques du liquide due à la température. A cet égard, il est important de noter que la dégradation de vitamines ou d'autres éléments nutritifs est une fonction de la température et du temps pendant lequel le liquide est maintenu à cette température.

Par rapport aux procédés thermiques tels que la pasteurisation, qui nécessite normalement une température de plus de 90° C pour certains liquides essentiellement aqueux pendant environ 60 secondes, le procédé selon l'invention, est très avantageux puisque la stérilisation nécessite moins d'une seconde à une température d'environ 62 à 75° C. Les effets de la température ne dépendent que de l'efficacité des phases d'échauffement et particulièrement de refroidissement.

La station de stérilisation 6 comporte une source d'énergie électrique pour alimenter un élément produisant un champ électrique ou électromagnétique pouvant générer un champ électrique d'environ 10³ V/cm dans le liquide 2 traversant la station de stérilisation. Dans l'exemple de la Fig. 1, le champ électrique est produit par un élément d'induction 9 qui, d'une part, chauffe le liquide et, d'autre part, applique un champ électrique d'une amplitude de l'ordre de grandeur 10³ V/cm. L'échauffement par induction du liquide nécessite que ce liquide soit conducteur, tel que de l'eau contenant des électrolytes, notamment des sels, même en faible quantité. L'échauffement par induction nécessite aussi une source de courant alternatif, de préférence à haute fréquence, par exemple de l'ordre de grandeur de 10⁶ Hz ou plus. La station de stérilisation 6 comporte en outre un capteur de température 10 relié à un circuit de contrôle 11 de la source de courant 1 pour régler l'apport d'énergie électrique et donc de l'échauffement du liquide.

A titre d'exemple, pour chauffer un litre d'eau en 0,3 sec de 30° C à 70° C, il faut avoir une puissance d'environ 500 kW.

Pour assurer une distribution de température homogène dans le liquide à stériliser, le système cinématique 4 porte un dispositif de rotation des récipients 3 qui permet d'une part, de mélanger le liquide à l'intérieur du récipient et, d'autre part de tourner le liquide dans le champ électrique ou électromagnétique. Le récipient 3 traverse la station de stérilisation 6 pendant sa rotation, de sorte que le champ électrique ou électromagnétique est appliqué de façon homogène sur tout le liquide qui traverse le générateur de champ 9. En raison de la très courte durée de stérilisation, la rotation peut s'effectuer de préférence à environ 1000 t/min.

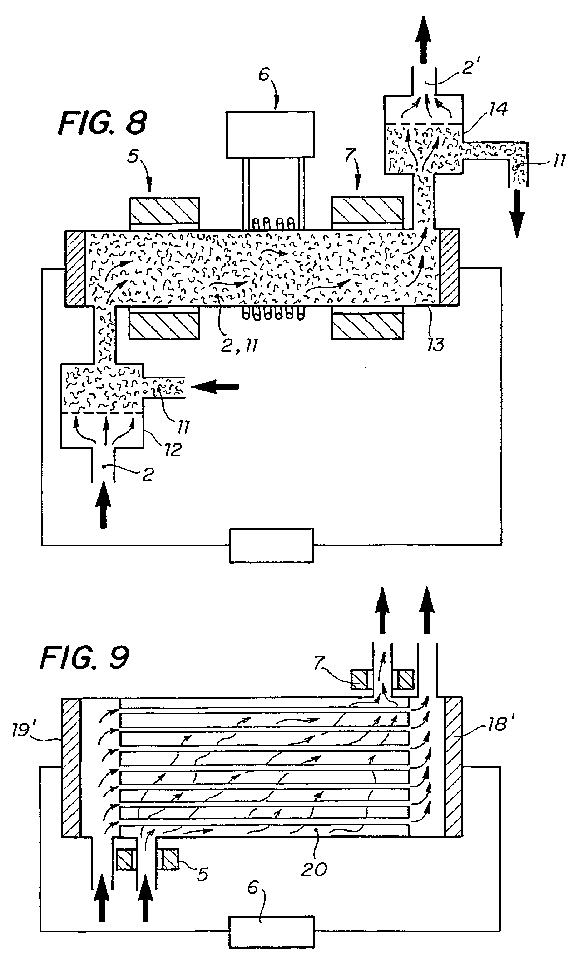

D'autres mouvements peuvent être appliqués au récipient 3 pour augmenter le transfert de chaleur par convection forcée à l'intérieur du récipient. Le principe du dispositif et du procédé décrit ci-dessus, peut aussi être appliqué à un liquide qui n'est pas dans un récipient hermétique, mais par exemple dans une conduite traversant le dispositif, le liquide s'écoulant en continu à travers cette conduite et pouvant être agité ou mis en rotation par des aubes ou autres éléments mécaniques dans la conduite agissant sur l'écoulement du liquide à travers cette conduite. Dans un tel système, il est possible d'ajouter des additifs 11, par exemple des billes en verre, ou une poudre chimiquement neutre et indissoluble, dans un mélangeur 12 avec le liquide à stériliser 2, ce qui permet de modifier l'inertie thermique moyenne du liquide, notamment la réduire, de façon à pouvoir chauffer et refroidir le liquide plus rapidement. Ceci est illustré à la Fig. 8 qui montre schématiquement les stations d'échauffement 5 et de refroidissement 7, ainsi que la station de stérilisation 6 disposées le long d'une conduite 13 amenant le liquide à stériliser 2 mélangé avec l'additif 11. En aval de la station de refroidissement 7, il y a une station de séparation 14, par exemple un filtre, qui sépare le liquide stérilisé 2' de l'additif 11.

Au lieu d'avoir un élément à induction pour l'échauffement et l'application du champ électrique, on peut aussi fournir des micro-ondes qui sont absorbées par le liquide 2 traversant un guide d'ondes 15, tel que montré dans la Fig. 2. Le récipient 3 traverse, par des trous 16, 17, le guide d'ondes 15 orthogonalement pour assurer que l'énergie des micro-ondes est absorbée par le liquide. La distance du centre du liquide du bout 18 du guide d'ondes 15 est un facteur à chiffre impair du quart de la longueur d'ondes (λ / 4, 3 λ / 4, 5 λ / 4 ...).

Dans la Fig. 3, une conduite 13 traverse le guide d'ondes 15 dans laquelle s'écoule un liquide à stériliser 2 ou, alternativement, une solution conductrice dans laquelle sont noyés des récipients 3' de forme quelconque et contenant le liquide à stériliser. De préférence, le liquide dans la conduite 13 a des propriétés similaires à celles du liquide à stériliser contenu dans le récipient 3'. Ceci permet de stériliser des récipients de petite taille et/ou de forme quelconque, par exemple ayant des emballages souples, le liquide dans la conduite 13 permettant un contrôle précis de la température et de l'application de champ électrique dans le liquide à stériliser.

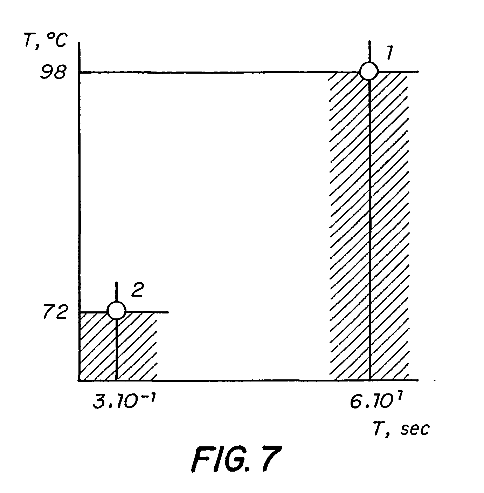

Outre l'élément d'induction 9 ou le guide d'ondes 15, le générateur de champ électrique ou électromagnétique peut avoir beaucoup de formes différentes, par exemple deux électrodes annulaires 18, 19 coaxiales, mais séparées par une certaine distance, tel que montré à la Fig. 4, ou une paire d'électrodes 18', 19' de part et d'autre du liquide à stériliser, tel que montre à la Fig. 5. Cette variante est bien adaptée aux récipients de forme parallélépipédique tels que les "briques" de lait ou de jus de fruit.

Dans la variante de la Fig. 6, le générateur de champ électrique ou électromagnétique comporte une électrode en forme de fil ou de tige constituant une électrode intérieure 18" autour de laquelle est placée, coaxialement, une électrode extérieure 19", l'espace entre les électrodes servant au passage du liquide à stériliser à travers une conduite 13". Dans les variantes des Figures 4 à 6, on peut appliquer un champ électrique généré par une tension continue entre les électrodes, le liquide étant amené à la température de stérilisation, c'est-à-dire de 60 à 75° C par la station d'échauffement 5, ou on peut appliquer une tension alternative à haute fréquence qui permet d'échauffer et simultanément d'appliquer le champ électrique au liquide.

La Fig. 9 montre une section d'un dispositif où l'application du champ électrique est effectuée par un système d'électrodes extérieures 18', 19', alimentées par une source d'électricité 6 à haute fréquence. Un échangeur de chaleur 20 permet de refroidir le liquide à stériliser 2 pendant la période d'application du champ électrique pour éviter un échauffement superflu du liquide 2 dû au passage du courant à haute fréquence à travers ce liquide.

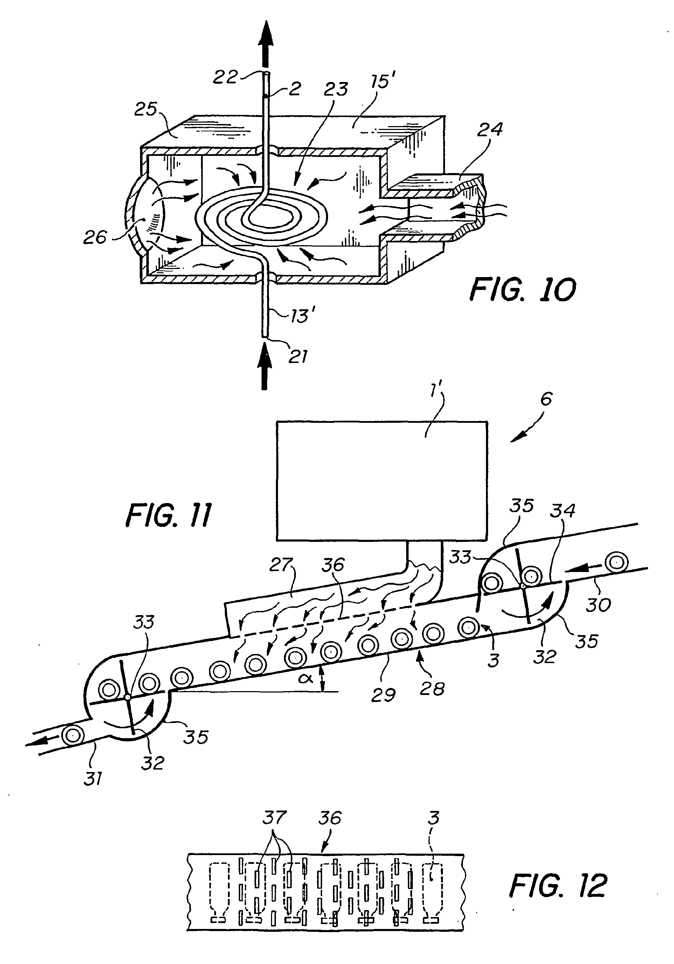

La Fig. 10 montre une partie d'une station de stérilisation utilisant des microondes, similaire à la variante de la Fig. 3, pour échauffer et stériliser un liquide à stériliser 2. Cette variante comprend un guide d'ondes 15' traversé par un conduit 13' dans lequel le liquide à stériliser 2 circule de bas 21 en haut 22.

Le guide d'ondes 15' comprend une partie d'entrée 24 et une partie d'enceinte 25 à l'intérieur de laquelle sont disposés ou formés un ou plusieurs réflecteurs 26. Ces réflecteurs peuvent être formés directement dans la paroi de la partie d'enceinte métallique 25, ou être montés en tant que pièces séparées à l'intérieur de la partie d'enceinte. Les réflecteurs peuvent être de forme généralement sphériques et servent à réfléchir les micro-ondes à l'intérieur de la partie d'enceinte 25 afin de distribuer ces micro-ondes de manière relativement uniforme dans le volume de l'enceinte.

Le conduit peut comporter une partie serpentin 23 disposée dans la partie d'enceinte 25 du guide d'ondes. Le conduit 13' peut être en un matériau absorbant faiblement la radiation micro-ondes, par exemple en quartz, en téflon ou en polyéthylène, de manière à ce que l'énergie des micro-ondes est principalement absorbée par le liquide à stériliser 2. Le liquide à stériliser se déplace dans le conduit 13' de bas en haut de manière à empêcher la formation et la stagnation de bulles gazeuses. La partie serpentin 23 permet d'accroître la longueur du conduit et donc d'augmenter le temps d'écoulement du liquide à stériliser à travers le guide d'ondes, de manière à ce que le dispositif soit très compact. Ceci permet également au conduit 13' d'avoir un faible diamètre afin d'échauffer, mais surtout de refroidir plus rapidement le liquide à stériliser.

La Fig. 11 montre une station de stérilisation 6 comprenant un générateur de micro-ondes 1' transmettant des micro-ondes par un guide d'ondes 27, et un système cinématique 28 pour des bouteilles ou autres récipients 3 remplies d'un liquide à stériliser 2 et fermées hermétiquement. Le système cinématique 28 comprend une partie de canal de traitement 29 s'étendant entre une partie de canal d'entrée 30 et une partie de canal de sortie 31. Les parties de canal d'entrée et de sortie sont munies de dispositifs d'écran contre les micro-ondes 32. Dans cette forme d'exécution, les dispositifs d'écran sont sous forme de tourniquets comprenant un axe de rotation 33 auquel sont fixées des pâles métalliques 34 à l'intérieur d'une partie de canal essentiellement cylindrique 35, dont le rayon est le même que la largeur des pâles 32 à part le jeu nécessaire pour la rotation des pâles. Les parties de canal d'entrée 30 et de sortie 31 débouchent sur la partie de canal essentiellement cylindrique, de manière à ce qu'elles ne sont pas en regard avec la partie de canal de traitement 29. Cette configuration permet de bloquer totalement et de manière fiable une fuite de micro-ondes.

Le guide d'ondes 27 est monté sur la partie de canal de traitement 29 et est séparé de cette partie par une paroi diffuseur 36 munie de fentes 37. La configuration des fentes est optimisée de manière à ce que le rayonnement des micro-ondes échauffe tout le volume du récipient essentiellement uniformément. La vitesse du passage des récipients dans la partie de canal de traitement 29 est également ajustée pour que le niveau de température de traitement du liquide à stériliser est atteint, en tenant compte de la vitesse de passage des récipients dans le canal ainsi que de la puissance de rayonnement des micro-ondes émises par le générateur 1'.

Les récipients 3 peuvent être déplacés par un système de convoyeurs (non illustré) à l'intérieur du canal. Dans une autre variante, pour le traitement de bouteilles essentiellement cylindriques, la partie de canal de traitement 29 peut être inclinée à un angle α permettant aux récipients de rouler librement le long du canal. Ce roulement permet non seulement d'éviter de fournir un mécanisme de déplacement, mais aussi de mélanger le liquide traité par convexion à l'intérieur du récipient. Ceci améliore l'uniformité de l'échauffement et donc du traitement par champ électrique.

L'invention est particulièrement effective pour tuer les micro-organismes de type moisissure, tels que *Aspergillus niger, Byssochlamys nivea* et *Byssochlamys fulva*, et des levures telles que *Saccharomyces cerevisiae*, aussi bien contenus dans le volume du liquide que présents préalablement sur les parois du récipient ou sur d'autres objets solides se trouvant dans le liquide. On ne peut détecter aucune modification des propriétés physico-chimiques du liquide. En particulier, le taux des vitamines, telles que la vitamine C, reste inchangé et d'autres qualités physico-chimiques déterminant le goût, l'odeur, la couleur ou les propriétés optiques du liquides ne sont pas influencées par la stérilisation selon l'invention.

On pense que l'effet de stérilisation selon l'invention repose sur les effets physiques suivants.

La structure des molécules lipides qui constituent la membrane enveloppant les micro-organismes est proche de celle des groupements de molécules d'eau appelées "clusters" et qui existent en tous milieux aqueux. L'interaction des structures dites "clusters" avec les molécules lipides est régie par les liaisons hydrogéniques. On peut modifier ces structures en les plaçant dans un champ électrique. Si ce champ est assez grand, il fait coïncider les topologies des structures, provoquant un affaiblissement local des liaisons entre les molécules lipides, entraînant des déchirures de la membrane des micro-organismes. C'est le principe de l'électroporation. Si le champ électrique est suffisamment élevé, les déchirures des membranes sont irréversibles et les micro-organismes sont détruits. L'amplitude du champ électrique doit néanmoins rester entre deux limites:
la limite supérieure définie par l'apparition de micro-arcs dans le liquide, c'est-à-dire la formation de zones de plasma qui détruisent non seulement les microorganismes mais modifient les propriétés physico-chimiques du liquide; et
la limite inférieure déterminée par une valeur du champ électrique insuffisante pour déchirer la membrane du micro-organisme de manière irréversible.

Un aspect important de l'invention est que la limite inférieure du champ peut être d'un ordre de grandeur de 10² V/cm si la température du liquide est plus grand que la température de seuil T_{S} d'un ordre de grandeur d'environ 60° à 75°C en l'absence de vibrations acoustiques. Les inventeurs pense qu'à cette température, il y a un effet de résonance entre les "clusters" d'eau et les lipides des membranes, de sorte que l'énergie nécessaire pour localement affaiblir les liaisons entre les molécules lipides peut être relativement faible du fait qu'elle est absorbée par les molécules en résonance au lieu d'être dissipées.

Il a été trouvé que la température de seuil T_{S} à laquelle la stérilisation s'effectue, dépend du micro-organisme mais se situe dans la gamme de températures de 62° à 75° C, en l'absence de vibrations acoustiques, pour des champs électriques d'environ 10² à 10³ V/cm, comme illustré dans le tableau ci-après.

| Microorganismes | T_{S} |
|---|---|
| Saccharomyces cerevisiae | 62° C |
| Aspergillus niger | 65 ° C |
| Byssochlamys nivea | 72° C |
| Byssochlamys fulva | 72° C |

Il est à noter que la durée d'application du champ électrique et de la température nécessaire pour la stérilisation peut être de moins d'une seconde, ce qui est très avantageux par rapport aux procédés conventionnels tels que la pasteurisation. On remarque aussi qu'il y a une corrélation entre le niveau de température atteint par le liquide, la durée du processus et le champ électrique présent.

Il a été également trouvé que la stérilisation peut être effectuée à une température inférieure à la température de seuil T_{S} par l'adjonction de vibrations acoustiques, notamment d'ultrasons, pendant le procédé de traitement. Les expériences faites dans le cadre de l'invention ont permis de démontrer que l'action des ultrasons renforce l'effet de destruction des microorganismes et permet de diminuer la température à laquelle le traitement s'effectue par une diminution ΔTᵤ d'environ 10 à 30° C. Les vibrations acoustiques font ajouter au mouvement chaotique des molécules du liquide dû à l'énergie thermique, un mouvement ondulatoire, de sorte que l'énergie nécessaire pour localement affaiblir les liaisons entre les molécules lipides est réduite.

La destruction d'un microorganisme par le champ électrique, par le procédé selon l'invention, nécessite une dépense d'énergie Δ. Dans le cas où le procédé selon l'invention est appliqué à un flux de produit liquide Φ_{L}, la puissance à dépenser N sera déterminée par la formule: $N = {N}_{a} + {N}_{T}$
où :
Nₐ est la puissance développée pour la destruction des microorganismes eux-mêmes, et
N_{T} est la puissance développée pour échauffer le produit de la température initiale T₀ à la température de seuil T_{S}.

Pour Nₐ, on a la relation: ${N}_{a} = {Φ}_{L} ⋅ X ⋅ Δ$
où :
Φ_{L} est le flux de produit traité et X est la concentration des microorganismes par unité de flux.

Pour N_{T} on a: ${N}_{T} = {Φ}_{L} + C ( {T}_{S} - {T}_{0} )$
où:
C est la capacité thermique spécifique moyenne du produit.

Dans le cas où le procédé selon l'invention est appliqué à un flux de containers hermétisés, remplis du produit à pasteuriser, on a : $N = {N}_{a} + {N}_{T}$
où : ${N}_{a} = V ⋅ X ⋅ Δ ⋅ ν$
et : ${N}_{T} = C V ( {T}_{S} - {T}_{0} ) ⋅ ν$
où:
ν est la fréquence avec laquelle les containers traversent la zone de champ électrique traitant,
V est le volume d'un container,
C est la capacité thermique spécifique moyenne de l'ensemble (container et liquide).

L'expérimentation a montré que, pour une concentration de microorganismes (levures, moisissures) inférieure à 10¹⁵ microorganismes/m³, la valeur optimale de X · Δ est $0 , 1 \leq X ⋅ Δ \leq 0 , 6 ( J / {cm}^{3} )$

Etant donné que l'effet des procédés selon l'invention est basé sur la coïncidence des topographies de deux structures, obtenues par l'ajustement opéré sur le champ électrique appliqué et le niveau de température, on peut s'attendre à avoir un effet sélectif, c'est-à-dire agissant uniquement sur les corps dont les structures sont en coïncidence, à savoir les micro-organismes et les clusters. Il n'y aura donc pas d'effet destructif ou modifiant sur les autres propriétés du liquide, ou sur les molécules qui y sont présentes. Après le traitement, le taux de vitamines (par exemple vitamine C) reste inchangé; les qualités physico-chimiques qui déterminent le goût, l'odeur, la couleur, les propriétés optiques et autres, ne sont pas influencées par le traitement.

Il est par contre possible, en utilisant le processus revendiqué, de procéder à l'inactivation des enzymes (contenues dans certains liquides comme par exemple les jus d'oranges) dont la structure, de nouveau, est similaire à celle des clusters de l'eau.

Dans ce cas, la pratique montre que, par exemple, pour un jus d'orange fraîchement pressé, T_{S} ≥ 70° C et ψ ≤ 2,5 (J/cm³), où ψ est la densité d'énergie nécessaire pour inactiver les enzymes.

De manière générale, les essais effectuées pour désinfecter différentes solutions aqueuses (jus de fruits et de légumes, limonades, lait et produits laitiers, sirops, bière, concentrés, boissons sportives, pâtes, purée, sauces et autres) ont montré, par exemple, qu'un traitement sous un champ électrique d'environ 10³ V/cm entre 60° C et 75° C pendant une durée de 0,3 seconde permet une réduction de 10⁹ de la concentration des moisissures (*Aspergillus niger, Byssochlamys nivea, Byssochiamys fulva),* et des levures *(Saccaromyces cerevisiae)* aussi bien contenues dans le volume du liquide traité que déposées préalablement sur les parois du récipient et sur des objets solides (morceaux de matériau PET, pulpe de fruits et de légumes). Le processus peut s'effectuer dans des flux stationnaires ou dynamiques.

La stérilisation du liquide a été confirmée pour des valeurs de l'amplitude du champ électrique appliqué allant de 10² V/cm à 10⁴ V/cm, que le courant électrique soit continu, alternatif, à basse, haute fréquence ou en micro-ondes.

Les échantillons ainsi désinfectés ont été conservés pendant plus de trois mois à température ambiante, Aucun indice de réversibilité, c'est-à-dire de réveil de l'activité des microorganismes détruits, n'a été observé. On peut donc parler d'une destruction irréversible des microorganismes.

Les avantages de cette invention sont considérables puisqu'ils permettent la stérilisation de liquides déjà remplis dans des récipients hermétiquement fermés, sans traitement de stérilisation au préalable du récipient ou du liquide. En outre, comparée à des procédés de pasteurisation conventionnels, la stérilisation selon l'invention est extrêmement rapide et permet de maintenir les propriétés physico-chimiques du liquide, donc d'éviter l'adjonction de suppléments de vitamines, chose courante dans l'industrie alimentaire. Les avantages sont aussi importants même si le liquide n'est pas contenu dans un récipient, par exemple un écoulement continu du liquide, notamment du fait que la stérilisation peut s'effectuer sans intervention dans le liquide, et à des débits importants.

Un autre avantage important est celui de pouvoir appliquer la stérilisation du liquide contenu dans des récipients tels que du PET et qui ne supportent pas les températures élevées de la pasteurisation.

A titre d'exemple, deux méthodes de désinfection d'une bouteille PET remplie d'un litre d'eau légèrement sucrée ou de jus de pomme contaminé par des moisissures "*Byssochlamys fulva*" à raison de 10⁹ micro-organismes/litre (bouteille) ont été comparées. La bouteille était fermée hermétiquement par un bouchon en polypropylène après remplissage. Le bouchon et la surface intérieure de la bouteille ont été contaminés à raison de 10⁶ microorganismes/cm² avant le remplissage. L'élimination des micro-organismes d'un ordre de grandeur de 10⁹ m.o./litre par pasteurisation a nécessité l'échauffement à 98° C pendant une minute. Dans un procédé selon l'invention, la stérilisation s'était effectuée par un échauffement à haute fréquence, appliquant un champ électrique d'environ 10³ V/cm pendant 0,3 sec à une température du liquide de 72° C résultant en la destruction de microorganismes d'un ordre de grandeur de 10⁹ m.o./litre.

La puissance à développer pour réaliser le procédé selon l'invention peut être calculée en utilisant les formules mentionnées ci-dessus, tel que montré dans les exemples suivants:

### Exemple 1 :

Produit traité : jus de pommes
Température initiale : T₀ = 20° C
Microorganismes à détruire : *Saccharomyces cerevisiae* et *Aspergillus niger*
Température de seuil: T_{S} = 65° C
Productivité de l'équipement : 1l/s
Type de flux : flux continu
Concentration des microorganismes : 10⁹ microorganismes/litre (m.o./l)
Capacité thermique moyenne : 4,2.10⁶.(J/m³.degré) $N {a}_{max} = {10}^{- 3} ( {m}^{3} / s ) ⋅ 0 , 6 ⋅ {10}^{3} ( kJ / {m}^{3} ) = 0 , 6 kW$ ${N}_{T} = {10}^{- 3} ( {m}^{3} / s ) ⋅ ( 65 - 20 ) degrés ⋅ 4 , 2 ⋅ {10}^{6} ⋅ ( J / {m}^{3} ⋅ degré ) = 189 kW$ ${N}_{max} = N {a}_{max} + {N}_{T} = 189 , 6 kW$

### Exemple 2 :

Idem à l'exemple 1, sauf que la température initiale est: T₀ = 60° C ${N}_{T} = {10}^{- 3} ( {m}^{3} / s ) ⋅ ( 65 - 60 ) degrés ⋅ 4 , 2 ⋅ {10}^{6} ⋅ ( J / {m}^{3} ⋅ degré ) = 21 kW$ $N {a}_{max} = 0 , 6 kW$ ${N}_{max} = 21 + 0 , 6 = 21 , 6 kW$

### Exemple 3 :

Produit traité : bouteilles PET (0,31) contenant du jus d'oranges avec pulpe
Température initiale : T₀ = 20° C
Microorganismes à détruire : *Byssochlamys fulva*
Température de seuil : T_{S} = 75° C
Productivité de l'équipement : 3 bouteilles/s
Concentration des microorganismes : 10⁹ m.o./l
Capacité thermique moyenne : 4,5 .10⁶.(J/m³. degré) $N {a}_{max} = 0 , 9 ⋅ {10}^{- 3} ( {m}^{3} / s ) ⋅ 0 , 6 ⋅ {10}^{3} ( kJ / {m}^{3} ⋅ degré ) = 0 , 54 kW$ ${N}_{T} = 0 , 9 ⋅ {10}^{- 3} ( {m}^{3} / s ) ⋅ ( 75 - 20 ) degrés ⋅ 4 , 5 ⋅ {10}^{6} ⋅ ( J / {m}^{3} ⋅ degré ) = 222 , 75 kW$ ${N}_{max} = 223 , 29 kW$

### Exemple 4 :

Idem à l'exemple 3, avec récupération thermique de 95% (par échangeur de chaleur) ${N}_{T} = 222 , 75 ⋅ ( 1 - 0 , 95 ) = 11 , 15 kW$ ${N}_{max} = 11 , 69 kW$

Un procédé selon l'invention a été testé pour différents types de microorganismes et à différentes températures pour déterminer le seuil de température nécessaire pour une stérilisation effective, le résultat étant présenté dans les exemples suivants:

### Exemple 5 :

Désinfection de solutions aqueuses contaminées par différents types de microorganismes.
Récipient: récipient PET 0,1 litre, préalablement stérile
   hauteur: 1,5 cm
   diamètre: 10 cm
Source de courant: HF 13,56 MHz. Nₘₐₓ = 60 kW
Durée du processus: τ = 0,3 sec.
Amplitude du champ électrique : 10³ V/cm
Niveau initial de contamination: entre 7 · 10 ⁸ et 1,2 · 10⁹ m.o./100 ml

La méthode de comptage du nombre de micro-organismes (m.o.) survivants a été effectuée selon des méthodes standards utilisées dans le domaine de la microbiologie.

| | **Concentration des micro-organismes survivants** | | | |
|---|---|---|---|---|
| **T** | ***Saccharomyces cerevisiae*** | ***Aspergillus niger*** | ***Byssochlamys nivea*** | ***Byssochlamys fulva*** |
| 20 | 7,5·10⁸ | 7,5·10⁸ | 7,1·10⁸ | 9,2·10⁸ |
| 40 | 7·10⁸ | 8·10⁸ | 7,5·10⁸ | 1,2·10⁹ |
| 45 | 6,2·10⁸ | 5·10⁸ | 8·10⁸ | 7·10⁸ |
| 50 | 3,4·10⁷ | 1,2·10⁶ | 3,5·10⁸ | 6·10⁸ |
| 55 | 7·10⁵ | 3·10⁴ | 7·10⁷ | 9·10⁷ |
| 60 | 5·10³ | 1,1·10¹ | 3·10⁶ | 3·10⁶ |
| 65 | <10⁰ | <10⁰ | 3·10³ | 8·10³ |
| 70 | <10⁰ | <10⁰ | 5·10⁰ | 2·10¹ |
| 75 | <10⁰ | <10⁰ | <10⁰ | <10⁰ |
| 80 | <10° | <10° | <10° | <10° |

D'après les résultats ci-dessus on constate que la stérilisation du liquide est complète (réduction de m.o. d'un facteur logarithmique de 9) à des températures entre 65 et 75° C, dépendant du type de micro-organisme.

### Exemple 6:

Désinfection de récipients, contaminés par différents types de micro-organismes
Récipient: récipient PET 0,1 litre, hermétiquement fermé
Liquide: jus de cassis, préalablement stérile
Source de courant: HF 13,56 MHz. Nₘₐₓ = 60 kW
Durée du processus: τ = 0,3 sec.
Amplitude du champ électrique: ~ 3 kV/cm
Niveau initial de contamination: 1,2 · 10⁶ - 3 · 10⁶ m.o./cm²
Quantité d'impulsions: 2 (2 positions du récipient)

Les valeurs données ci-après sont la somme des résultats d'un
a) comptage des m.o. survivants dans le liquide, et d'un
b) comptage du nombre de m.o. survivants sur la surface du récipient et du bouchon

| **T** | ***Saccharomyces cerevisiae*** | ***Aspergillus niger*** | ***Byssochlamys nivea*** | ***Byssochlamys fulva*** |
|---|---|---|---|---|
| 20 | 1,4·10⁸ | 2,3·10⁸ | 1,5·10⁸ | 7,5·10⁷ |
| 40 | 1,2·10⁸ | 2,1·10⁸ | 9,1·10⁷ | 7,1·10⁷ |
| 45 | 7,4·10⁷ | 1,8·10⁸ | 1,2·10⁸ | 6,3·10⁷ |
| 50 | 2,2·10⁵ | 1,4·10⁶ | 1,1·10⁷ | 9,1·10⁶ |
| 55 | 4,6·10³ | 2,1·10⁵ | 7,7·10⁶ | 1,0·10⁶ |
| 60 | 1,2·10¹ | 5,5·10² | 3,0·10⁵ | 9,0·10⁴ |
| 65 | <10⁰ | 1,9·10⁰ | 4,1·10³ | 2,2·10² |
| 70 | <10⁰ | <10⁰ | 1,2·10⁰ | 4,1·10¹ |
| 75 | <10⁰ | <10⁰ | <10⁰ | <10⁰ |
| 80 | < 10⁰ | < 10⁰ | <10⁰ | <10⁰ |

On constate que la stérilisation de la surface du récipient est complète (réduction - 9 log) à des températures entre 65 et 75° C. Cette température dépend du type de microorganisme.

### Exemple 7 :

Désinfection de pulpe dans un jus d'orange
Récipient: récipient PET, 0,1 litre
Liquide: jus d'orange avec pulpe
Source de courant: micro-onde 915 MHz. Nₘₐₓ = 60 kW
Durée du traitement: τ = 0,5 sec.
Amplitude du champ électrique: ~ = 10³ V/cm
Niveau initial de contamination: 6 · 10⁷ m.o./100 ml

Les valeurs dans le tableau ci-après sont le résultat du comptage du nombre de m.o. survivants dans le liquide + pulpe

### Saccharomyces cerevisiae

| **T** | **Nombre de microorganismes survivants, m.o/100ml** |
|---|---|
| 20 | 6·10⁷ |
| 40 | 5·10⁷ |
| 45 | 2,5·10⁷ |
| 50 | 4,1·10⁵ |
| 55 | 3,2·10³ |
| 60 | 4,1·10¹ |
| 65 | <10⁰ |
| 70 | < 10⁰ |
| 75 | <10⁰ |
| 80 | <10⁰ |

La stérilisation du jus d'orange et de la pulpe est complète (réduction ~ 8 log) à la température de 65° C.

### Exemple 8:

Comparaison du procédé proposé avec la pasteurisation classique *(Byssochlamys fulva)* Conditions du procédé proposé:
Récipient: récipient PET 0,1 litre
Liquide: jus de pommes contaminé par *Byssochlamys fulva*
Source de courant: HF, 13,56 MHz, Nₘₐₓ = 60 kW
Durée du traitement: τ = 0,3 sec.
Amplitude du champ électrique: ~ 10³ V/cm
Niveau initial de contamination: 7,5 · 10⁸ m.o./100 ml
Conditions de pasteurisation: standard
Durée de traitement: 60 sec.
Méthode de numération: comptage du nombre de m.o. survivants dans le liquide

| | ***Byssochlamys fulva*** | |
|---|---|---|
| **T** | **Procédé proposé dans la présente invention** | **Pasteurisation standard** |
| 20 | 9,2·10⁸ | 7,6·10⁸ |
| 65 | 8·10³ | 7,1·10⁸ |
| 70 | 2·10¹ | 6,8·10⁷ |
| 75 | <10⁰ | 7,0·10⁶ |
| 80 | <10⁰ | 6,4·10⁴ |
| 85 | <10⁰ | 8,2·10² |
| 90 | <10⁰ | 5,9·10¹ |
| 95 | <10⁰ | 3·10⁰ |
| 100 | <10⁰ | <10⁰ |

La stérilisation par le procédé selon l'invention permet de diminuer la température et de réduire la durée de plusieurs ordres de grandeur, comparé aux procédés de pasteurisation standard. Ce résultat est illustré dans le tableau suivant:

| **Processus** | **Température finale (°C)** | **Durée (s)** |
|---|---|---|
| Procédé revendiqué | 73 | 1 |
| Pasteurisation standard | 98 | 60 |
| Pasteurisation standard | 88 | 600 |

### Exemple 9:

Influence de la quantité d'énergie électromagnétique appliquée, de la température initiale et de la température finale sur la qualité du processus revendiqué (cas des m.o. *Saccharomyces cerevisiae)*.
Milieu: eau + 0,5 g/l de NaCl
(densité: 1g/cm³; capacité thermique c= 4,18 J/g.°C)
Micro-organismes: *Saccharomyces cerevisiae*
   Concentration initiale: (1,4 - 5,1) · 10⁸m.o./100 ml
Source de courant: HF 13,56 MHz
Volume de la cellule de traitement: 100 ml
Résultats des mesures:

### Saccharomyces cerevisiae

| **Processus** | **Température initiale °C** | **Energie appliquée J/g** | **Température finale °C** | **Durée s** | **Nombre de microorganismes survivants mo/100ml** |
|---|---|---|---|---|---|
| Procédé revendiqué | 4 | 257,3 | 65,3 | <1 | <10° |
| Procédé revendiqué | 20 | 191,1 | 65 | <1 | <10° |
| Procédé revendiqué | 40 | 105,1 | 65,3 | <1 | <10° |
| Procédé revendiqué | 59,7 | 28,5 | 65,4 | <1 | <10° |
| Procédé revendiqué | 62,1 | 13 | 65,3 | <1 | 4100 |
| Procédé revendiqué | 61,2 | 24 | 67,1 | <1 | <10° |
| Pasteurisation standard | - | - | 78 | 60 | <10° |

L'énergie que doit recevoir le liquide pour qu'il atteigne l'état critique de résonance qui correspond au seuil de stérilisation complète, se communique au liquide par deux canaux:
1. par échauffement du liquide (celui-ci peut être un échauffement par convection, un échauffement ohmique, etc.)
2. par l'action non-ohmique du champ électromagnétique, qui crée l'effet de résonance.

La densité d'énergie de seuil Es du seuil du champ électromagnétique répond à la condition 12,0<Es<24,0 J/g pour les micro-organismes *Saccharomyces cerevisiae*.

On voit qu'il y a une température seuil Ts de désinfection par champ électrique ou électromagnétique, proche de la valeur de 65° C pour les levures *Saccharomyces cerevisiae.*

### Exemple 10:

Influence de la quantité d'énergie électromagnétique appliquée, de la température initiale et de la température finale, sur la qualité du processus revendiqué (cas des m.o. *Byssochlamys fulva*).
Milieu: eau + 0,5 g/l de NaCl
   ρ = 1g/cm³; c= 4,18 J/g.° C
Micro-organismes: *Byssochlamys fulva*
   Concentration initiale : (1,7 - 4,5) · 10⁸ m.o/100 ml
Source de courant: HF 13,56 MHz
Volume de la cellule de traitement: 100 ml

### Résultats des mesures:

### Byssochlamys fulva

| **Processus** | **Température initiale °C** | **Énergie du champ électromagnétique J/g** | **Température finale °C** | **Durée S** | **Nombre de m.o. survivants m.o./100ml** |
|---|---|---|---|---|---|
| Procédé revendiqué | 4,5 | 288,8 | 73,4 | <1 | <10° |
| Procédé revendiqué | 19,8 | 222,9 | 73 | <1 | <10° |
| Procédé revendiqué | 40,1 | 138,6 | 73,2 | <1 | <10° |
| Procédé revendiqué | 55,2 | 75,4 | 73,4 | <1 | <10° |
| Procédé revendiqué | 65,1 | 34,6 | 73,3 | <1 | <10° |
| Procédé revendiqué | 70,2 | 12,1 | 73 | <1 | 12000 |
| Procédé revendiqué | 70,3 | 34,3 | 78,5 | <1 | <10° |
| Pasteurisation standard | - | - | 98 | 60 | <10° |

On voit qu'il existe une température seuil Ts de désinfection par champ électrique ou électromagnétique, proche de valeur de 75° C pour les moisissures *Byssochlamys fulva*.
La densité d'énergie de seuil Es du champ électromagnétique dans le cas des m.o. *Byssochlamys fulva* répond à la condition: Es<34,3 J/g

### Exemple 11:

Influence du paramètre ρc (ρ, densité du liquide et c, capacité thermique du liquide) sur la qualité du processus revendiqué (le paramètre pc caractérise l'inertie thermique du liquide)
Milieu: référence: eau + 0,5 g/l de NaCl (cₒ = 4,19 J/g.° C; ρₒ = 1 g/cm³)
   milieu: 1) inertie thermique réduite par rapport à la référence (ρᵢcᵢ): différentes solutions de concentré d'orange dans l'eau
   2) inertie thermique augmentée par rapport à la référence: différentes solutions aqueuses de purée de banane.
Micro-organismes: *Saccharomyces cerevisiae*
   Concentration initiale: (0,1 à 5) · 10⁻⁸ m.o/100 ml
Source de courant: HF 13,56 MH7
Volume de la cellule testée: 100 ml

### Saccharomyces cerevisiae

| **Objet du traitement** | **pc** | **Température initiale °C** | **Énergie appliquée J/g** | **Température finale °C** | **Durée s** | **Nombre de m.o. survivants /100ml** |
|---|---|---|---|---|---|---|
| 100 % de concentré d'orange | 0,7 | 20 | 132,9 | 66,7 | 0,5 | 530 |
| 100 % de concentré d'orange | 0,7 | 20 | 139,4 | 67,6 | 0,3 | 60 |
| 100 % de concentré d'orange | 0,7 | 20 | 142,7 | 68,9 | 0,4 | <10° |
| 33 % d'eau + 67 % de concentré d'orange | 0,8 | 20 | 153,4 | 66,1 | 0,4 | 870 |
| 33 % d'eau + 67 % de concentré d'orange | 0,8 | 20 | 171,3 | 67,5 | 0,2 | <10° |
| 67 % d'eau + 33 % de concentré d'orange | 0,9 | 20 | 169,1 | 65,4 | 0,3 | 4300 |
| 67 % d'eau + 33 % de concentré d'orange | 0,9 | 20 | 175,2 | 66,1 | 0,4 | <10° |
| 100 % d'eau + 0,5 g/l de NaCl | 1 | 20 | 191,6 | 65,4 | 0,3 | <10° |
| 67 % d'eau + 33 % de purée de banane | 1,07 | 20 | 196,9 | 63,5 | 0,4 | <10° |
| 33 % d'eau + 67 % de purée de banane | 1,15 | 20 | 200,5 | 61,3 | 0,2 | <10° |
| 100 % de purée de banane | 1,24 | 20 | 191,8 | 57 | 0,3 | 1400 |
| 100 % de purée de banane | 1,24 | 20 | 204,7 | 59,3 | 0,3 | <10° |

### Exemple 12:

Stérilisation d'un flux continu de jus de pommes contaminé par des levures du type *Saccharomyces cerevisiae* en appliquant des vibrations acoustiques (ultrasons) lors du traitement.
Débit du liquide traité: 1 litre/minute
Source de courant: HF 2,45 GHz, Puissance = 1,5 kW
Durée du traitement: τ = 1 sec.
Niveau initial de concentration de levures: 1·10⁶ CFU/cm³
Frequences des ultrasons appliqués: 22KHz, Puissance 0,6KW
Température initiale: 20°C

Les valeurs données ci-après sont la somme des résultats d'un comptage des m.o. survivants dans le liquide pour un traitement avec ultrasons et sans ultrasons à titre de comparaison:

### Nombre de microorganismes survivants, m.o./ml

| **Température, °C** | **Pasteurisation standard** | **Procédé sans application des ultrasons** | **Procédé avec application des ultrasons** |
|---|---|---|---|
| 20 | 6.10⁷ | 6.10⁷ | 6,1.10⁷ |
| 40 | 5,9.10⁷ | 5,1.10⁷ | 5,4.10⁶ |
| 45 | 5,7.10⁷ | 2,5.10⁷ | 8,7.10⁴ |
| 50 | 4,3.10⁶ | 4,1.10⁵ | 4,1.10² |
| 55 | 1,8.10⁵ | 1,6.10³ | < 10⁰ |
| 60 | 8,9.10³ | 4,1.10¹ | < 10⁰ |
| 65 | 5,4.10² | < 10⁰ | < 10⁰ |
| 70 | < 10⁰ | < 10⁰ | < 10⁰ |
| 75 | < 10⁰ | < 10⁰ | < 10⁰ |

### Exemple 13:

Stérilisation d'un flux continu de jus de cassis contaminé par des moisissures du type *Byssochlamys nivea* en appliquant des vibrations acoustiques (ultrasons) lors du traitement.
Débit du liquide traité: 2 litre/minute
Source de courant: HF 13,56 MHz, Puissance = 1 kW
Durée du traitement: τ = 0,7 secondes.
Niveau initial de concentration de moisissures: 7,5·10⁷ CFU/cm³
Frequences des ultrasons appliqués: 40KHz, Puissance 0,4KW
Température initiale: 20°C

Les valeurs données ci-après sont la somme des résultats d'un comptage des m.o. survivants dans le liquide pour un traitement avec ultrasons et sans ultrasons à titre de comparaison:

| **Température, °C** | **Pasteurisation standard** | **Procédé sans application des ultrasons** | **Procédé avec application des ultrasons** |
|---|---|---|---|
| 20 | 7,3.10⁷ | 7,5.10⁷ | 7,5.10⁷ |
| 40 | 7,3.10⁷ | 7,1.10⁷ | 3,2.10⁷ |
| 45 | 7,3.10⁷ | 6,3.10⁷ | 7,1.10⁶ |
| 50 | 7,3.10⁷ | 9,1.10⁶ | 5,3.10⁵ |
| 55 | 6,9.10⁷ | 1,0.10⁶ | 2,4.10⁴ |
| 60 | 4,1.10⁷ | 9,0.10⁴ | 3,8.10³ |
| 65 | 7,6.10⁶ | 2,2.10² | 5,9.10¹ |
| 70 | 5,3.10⁵ | 4,1.10¹ | < 10⁰ |
| 75 | 4,2.10⁴ | < 10⁰ | < 10⁰ |
| 80 | 3,4.10³ | < 10⁰ | < 10⁰ |
| 85 | 8,9.10¹ | < 10⁰ | < 10⁰ |
| 90 | 3,0.10⁰ | < 10⁰ | < 10⁰ |
| 95 | < 10⁰ | < 10⁰ | < 10⁰ |

D'après ces résultats, on constate que:
· La température critique de stérilisation par champ électrique augmente quand l'inertie thermique du milieu diminue, et vice versa, diminue quand l'inertie thermique du milieu augmente.
   L'adjonction de vibrations acoustiques pendant un procédé de traitement de stérilisation selon l'invention, renforce l'effet de destruction des microorganismes et permet de diminuer la température à laquelle le traitement s'effectue.
· Les expériences exécutées permettent de mettre en oeuvre un procédé de contrôle de la température de stérilisation du liquide traité par champ électromagnétique et le dispositif correspondant en ajoutant au liquide des additifs, permettant soit d'augmenter soit de diminuer la température critique de stérilisation par champ électromagnétique.
· En particulier, on pourra augmenter ou diminuer l'inertie thermique du liquide traité en ajoutant un échangeur de chaleur, refroidissant le liquide traité dans la zone d'application du champ électromagnétique.
· Pour conserver le maximum de vitamines dans la boisson traitée, il y aura lieu d'exécuter le processus de stérilisation électromagnétique dans un milieu ayant la plus grande valeur possible du coefficient d'inertie thermique. Par exemple, la température critique d'un jus d'orange sera diminuée quand on ajoute à ce jus de la pulpe. Cette pulpe peut après le traitement électromagnétique, être filtrée.

On peut aussi utiliser des additifs sous forme de suspension de haute inertie thermique (globules contenant un liquide qui passe d'une phase à l'autre à la température critique).

## Revendications

1. Procédé de stérilisation d'un liquide et/ou d'un objet solide plongé dans un liquide, comportant l'étape ou les étapes d'échauffement d'un liquide à une température de traitement inférieure à une température nécessaire pour la stérilisation par pasteurisation, et d'application d'un champ électrique de 10² V/cm à 10⁴ V/cm, **caractérisé en ce que** l'on soumet le liquide et/ou l'objet à des vibrations acoustiques pendant l'application du champ électrique.

2. Procédé selon la revendication 1, **caractérisé en ce que** les vibrations acoustiques ont des fréquences dans le domaine des ultrasons.

3. Procédé selon l'une des revendications précédentes, **caractérisé en ce que** la température de traitement est égale ou supérieure à une température de seuil Tₛ moins une diminution de température ΔTᵤ dépendant de l'application de vibrations acoustiques, la température de seuil Tₛ se trouvant dans la gamme de températures allant de 60 à 75°C.

4. Procédé selon la revendication précédente, **caractérisé en ce que** et la diminution de température ΔTᵤ se trouve dans la gamme de températures allant de 0 à 30°C.

5. Procédé selon l'une des revendications précédentes, **caractérisé en ce que** l'énergie apportée par ledit champ électrique est intérieure à 0,6 J/cm³, mais supérieure à 0,1 J/cm³.

6. Procédé selon l'une des revendications précédentes, **caractérisé en ce que** l'on chauffe le liquide à une température de seuil Tₛ supérieure à environ 70° C et que l'on applique un champ électrique produisant une densité d'énergie inférieure à 2,5 J/cm³ pour effectuer l'inactivation d'enzymes.

7. Procédé selon l'une des revendications précédentes, **caractérisé en ce que** le liquide est échauffé essentiellement uniformément à la température de traitement en moins de trois secondes.

8. Procédé selon l'une des revendications précédentes, **caractérisé en ce que** le champ électrique est généré par la même source d'énergie réchauffant le liquide.

9. Procédé selon l'une des revendications précédentes, **caractérisé en ce que** le champ électrique et l'échauffement sont produits par un rayonnement électromagnétique de type micro-ondes.

10. Procédé selon l'une des revendications 1 à 8, **caractérisé en ce que** l'échauffement du liquide est produit par effet inductif à basse fréquence.

11. Procédé selon l'une des revendications 1 à 8, **caractérisé en ce que** le champ électrique est produit par un champ électrique unipolaire.

12. Procédé selon l'une des revendications 1 à 8, **caractérisé en ce que** le champ électrique est produit par un champ électrique continu.

13. Procédé selon l'une des revendications précédentes, **caractérisé en ce que** le procédé de stérilisation est appliqué à des bouteilles hermétiquement fermées et contenant un liquide à stériliser.

14. Procédé selon la revendication précédente, **caractérisé en ce que** la bouteille est tournée autour de son axe à une vitesse de 1000 t/min ou plus.

15. Procédé selon l'une des revendications précédentes, **caractérisé en ce que** le récipient est soumis à l'action de plusieurs impulsions de champ électrique, chaque impulsion correspondant à une position différente d'un espace gazeux contenu dans le container.

16. Procédé selon l'une des revendications précédentes, **caractérisé en ce que** le procédé est appliqué à des objets solides plongés dans, ou en contact avec un liquide.

17. Procédé selon l'une des revendications précédentes, **caractérisé en ce que** l'inertie thermique du liquide à stériliser est modifiée par l'adjonction d'éléments de matériaux diélectriques ayant une capacité thermique moyenne plus élevée que le liquide à stériliser.

18. Procédé selon l'une des revendications précédentes, **caractérisé en ce que**, après l'application du champ électrique, le liquide est refroidi par un échangeur de chaleur couplé à un dispositif de pré-chauffage en amont d'un dispositif d'application du champ électrique.

19. Procédé selon l'une des revendications précédentes, **caractérisé en ce que** la durée d'exposition dudit liquide au champ électrique est inférieure à une seconde.

20. Dispositif pour la mise en oeuvre du procédé selon l'une des revendications précédentes, **caractérisé en ce que** le dispositif comporte un système cinématique (4) pour le positionnement et le déplacement du produit de liquide (2) et une station de stérilisation (6) disposée le long du système cinématique (4), la station de stérilisation comportant une source d'énergie électrique pour produire un champ électrique de 10² V/cm à 10⁴ V/cm dans le liquide traversant la station de stérilisation et un générateur d'ultrasons.

21. Dispositif selon la revendication précédente, **caractérisé en ce qu'**il comporte une station d'échauffement (5) en amont de la station de stérilisation.

22. Dispositif selon la revendication précédente, **caractérisé en ce qu'**il comporte une station de refroidissement (7) en aval de la station de stérilisation.

23. Dispositif selon la revendication précédente, **caractérisé en ce que** les stations d'échauffement et de refroidissement sont munies d'un échangeur de chaleur pour récupérer la chaleur de la station de refroidissement et l'utiliser pour échauffer le liquide dans la station d'échauffement.

24. Dispositif selon l'une des revendications 20 à 23, **caractérisé en ce que** la source d'énergie électrique a une puissance apte à pouvoir chauffer le liquide à stériliser à au moins 60° C et à produire un champ électrique dans le liquide à stériliser d'environ 10³ V/cm.

25. Dispositif selon l'une des revendications 20 à 23, **caractérisé en ce que** la source d'énergie électrique est un élément d'induction produisant un champ électromagnétique.

26. Dispositif selon l'une des revendications 20 à 23, **caractérisé en ce que** la source d'énergie électrique comporte au moins une paire d'électrodes disposées de part et d'autre du liquide à stériliser et aptes à créer un champ capacitif.

27. Dispositif selon l'une des revendications 20 à 23, **caractérisé en ce que** la source d'énergie électrique est un générateur de micro-ondes.

28. Dispositif selon la revendication précédente, **caractérisé en ce que** la station de stérilisation comprend un guide d'ondes relié au générateur de micro-ondes, le guide d'ondes comportant une partie d'enceinte (25) dans laquelle sont disposés un ou plusieurs réflecteurs d'ondes courbes (26), la station de stérilisation comprenant en outre un conduit (13') dans lequel s'écoule le liquide à stériliser (2) à travers la partie d'enceinte (25) de bas en haut.

29. Dispositif selon la revendication précédente, **caractérisé en ce que** le conduit 13' comprend une partie serpentin disposée à l'intérieur de la partie d'enceinte du guide d'ondes.

30. Dispositif selon l'une des revendications 20 à 27, **caractérisé en ce qu'**il comprend, en amont de la station de stérilisation, un mélangeur permettant d'ajouter des additifs augmentant ou diminuant l'inertie thermique moyenne du liquide traité et, en aval, une station de séparation permettant de séparer le liquide traité dudit additif.

31. Dispositif selon l'une des revendications 20 à 28, **caractérisé en ce que** la station de stérilisation comporte un échangeur de chaleur permettant de limiter la température d'échauffement du liquide traité.

32. Dispositif selon la revendication 27, **caractérisé en ce que** la station de stérilisation comprend une partie de canal de traitement (29) dans laquelle se déplacent les récipients (3) remplis de liquide à stériliser, la partie de canal de traitement étant reliée à un guide d'ondes (27) du générateur de micro-ondes (1') par une paroi munie de fentes (37).

33. Dispositif selon la revendication précédente, **caractérisé en ce que** la partie de canal de traitement est munie en aval et en amont d'un dispositif d'écran à micro-ondes comprenant un tourniquet muni de pâles (32) bloquant les micro-ondes.

34. Dispositif selon la revendication 32 ou 33, **caractérisé en ce que** la partie de canal de traitement (29) est inclinée à un angle α permettant à des récipients de se déplacer le long de la partie de canal par la force de gravité.

## Claims

1. A method for sterilising a liquid and / or a solid object immersed in a liquid, including the step or the steps of heating a liquid to a treatment temperature lower than a temperature needed for sterilising by pasteurisation and of applying an electric field of an order of magnitude of about 10² V / cm or more, **characterised in that** the liquid or / and the object is / are subjected to acoustic vibrations during the application of the electric field

2. A method according to claim 1, **characterised in that** the acoustic vibrations have frequencies in the ultrasound domain.

3. A method according to one of the preceding claims, **characterised in that** the treatment temperature is equal or higher than a threshold temperature Tₛ, minus a temperature decrease ΔTᵤ which is dependent upon the application of acoustic vibrations, the threshold temperature Tₛ being situated approximately within the range of temperatures from 60 to 75 °C.

4. A method according to the preceding claim, **characterised in that** the temperature decrease ΔTᵤ is in the range of temperatures from 0 to 30 °C.

5. A method according to one of the preceding claims, **characterised in that** the energy supplied by said electric field is less than 0.6 J / cm³, but higher than 0.1 J /cm³.

6. A method according to one of the preceding claims, **characterised in that** the liquid is heated to a threshold temperature Tₛ higher than about 70 °C and **in that** an electric field is applied which produces an energy density less than 2.5 J / cm³, to ensure the inactivation of enzymes.

7. A method according to one of the preceding claims, **characterised in that** the liquid is heated substantially uniformly at a treatment temperature for less than three seconds.

8. A method according to one of the preceding claims, **characterised in that** the electric field is generated by the same source of energy as that heating the liquid.

9. A method according to one of the preceding claims, **characterised in that** the electric field and the heating are produced by electromagnetic radiations of the microwave type.

10. A method according to one of claims 1 to 8, **characterised in that** the heating of the liquid is produced by a low frequency induction effect.

11. A method according to one of claims 1 to 8, **characterised in that** the electric field is produced as a unipolar electric field.

12. A method according to one of claims 1 to 8, **characterised in that** the electric field is produced as a continuous electric field.

13. A method according to one of the preceding claims, **characterised in that** the sterilisation method is applied to hermetically closed bottles containing a liquid to be sterilised.

14. A method according to the preceding claim, **characterised in that** the bottle is rotated about its axis at a speed of about 1000 rpm or more.

15. A method according to one of the preceding claims, **characterised in that** the container is subjected to the action of several pulses of an electric field, each pulse corresponding to a different position of a gaseous space contained within the container.

16. A method according to one of the preceding claims, **characterised in that** the method is applied to solid objects which are immersed in a liquid or in contact therewith.

17. A method according to one of the preceding claims, **characterised in that** the thermal inertia of the liquid to be sterilised is modified by the addition of elements of dielectric materials having an average thermal capacity higher than the liquid to be sterilised.

18. A method according to one of the preceding claims, **characterised in that**, after the application of the electric field, the liquid is cooled by a heat exchanger coupled to a preheating device upstream of a device for applying the electric field.

19. A method according to one of the preceding claims, **characterised in that** the duration of the exposure of said liquid to the electric field is less than one second.

20. A device for carrying out the method according to one of the preceding claims, **characterised in that** the device includes a kinematic system (4) for the positioning and the moving of the liquid product (2) and a sterilisation station (6) positioned along the kinematic system (4), the sterilisation station including a source of electric energy for producing an electric field of about 10² V / cm to 10⁴ V / cm in the liquid travelling through the sterilisation station and an ultrasound generator.

21. A device according to the preceding claim, **characterised in that** it includes a heating station (5) upstream of the sterilisation station.

22. A device according to the preceding claim, **characterised in that** it includes a cooling station (7) downstream of the sterilisation station.

23. A device according to the preceding claim, **characterised in that** the heating and the cooling stations are provided with a heat exchanger for recovering the heat from the cooling station and using the same for heating the liquid in the heating station.

24. A device according to one of claims 20 to 23, **characterised in that** the source of electric energy has a power sufficient for heating the liquid to be sterilised to at least 60 °C and to produce an electric field in the liquid to be sterilised of about 10³ V/cm.

25. A device according to one of claims 20 to 23, **characterised in that** the source of electric energy is an induction element producing an electromagnetic field.

26. A device according to one of claims 20 to 23, **characterised in that** the source of electric energy includes at least one pair of electrodes, which are arranged one on each side of the liquid to be sterilised and which are capable of generating a capacitive field.

27. A device according to one of claims 20 to 23, **characterised in that** the source of electric energy is a microwave generator.

28. A device according to the preceding claim, **characterised in that** the sterilisation station includes a wave-guide connected to a microwave generator, the wave-guide including an enclosure part (25) in which is or are arranged one or more curved wave reflectors (26), the sterilisation station further including a conduit (13') in which flows the liquid (2) to be sterilised through the enclosure part (25), from its bottom to its top.

29. A device according to the preceding claim, **characterised in that** the conduit (13') includes a helical coil housed inside the enclosure part of the wave-guide.

30. A device according to one of claims 20 to 27, **characterised in that** it includes, upstream of the sterilisation station, a mixer which allows the introduction of additives which increase or decrease the average thermal inertia of the liquid treated and, downstream thereof, a separation station enabling a separation of the liquid treated from said additive.

31. A device according to one of claims 20 to 28, **characterised in that** the sterilisation station includes a heat exchanger, which makes it possible to limit the temperature to which the liquid treated is heated.

32. A device according to claim 27, **characterised in that** the sterilisation station includes a treatment channel part (29) in which the containers (3) filled with a liquid to be sterilised move, the treatment channel part being connected to a wave-guide (27) of the microwave generator (1') through a wall provided with slots (37)

33. A device according to the preceding claim, **characterised in that** the treatment channel part is provided, upstream and downstream thereof, with a screen device for the microwaves, including a turnstile carrying blades (32) for blocking off the microwaves.

34. A device according to claim 32 or 33, **characterised in that** the treatment channel part (29) slopes at an angle α enabling the containers to move along the channel part by the effect of the forces of gravity.

## Patentansprüche

1. Verfahren zur Sterilisation einer Flüssigkeit und/oder eines in die Flüssigkeit eingetauchten festen Gegenstandes mit dem Schritt bzw. den Schritten, eine Flüssigkeit auf eine Behandlungstemperatur zu erwärmen, die unter einer für die Sterilisation durch Pasteurisierung erforderlichen Temperatur liegt, und ein elektrisches Feld von 10² V/cm bis 10⁴ V/cm anzulegen, **dadurch gekennzeichnet, dass** die Flüssigkeit und/oder der Gegenstand während der Anwendung des elektrischen Feldes akustischen Schwingungen ausgesetzt werden.

2. Verfahren nach Anspruch 1, **dadurch gekennzeichnet, dass** die akustischen Schwingungen Frequenzen im Ultraschallbereich besitzen.

3. Verfahren nach einem der vorangehenden Ansprüche, **dadurch gekennzeichnet, dass** die Behandlungstemperatur gleich oder höher als eine Schwellentemperatur Tₛ, verringert um eine Temperaturverringerung ΔTᵤ ist, die von der Anwendung akustischer Schwingungen abhängt, wobei die Schwellentemperatur Tₛ im Temperaturbereich von 60 bis 75 °C liegt.

4. Verfahren nach dem vorangehenden Anspruch, **dadurch gekennzeichnet, dass** die Temperaturverringerung ΔTᵤ im Temperaturbereich von 0 bis 30 °C liegt.

5. Verfahren nach einem der vorangehenden Ansprüche, **dadurch gekennzeichnet, dass** die durch das elektrische Feld eingebrachte Energie kleiner als 0,6 J/cm³, aber grösser als 0,1 J/cm³ ist.

6. Verfahren nach einem der vorangehenden Ansprüche, **dadurch gekennzeichnet, dass** man die Flüssigkeit auf eine Schwellentemperatur Tₛ von mehr als etwa 70 °C erwärmt und ein elektrisches Feld anlegt, das eine Energiedichte von weniger als 2,5 J/cm³ erzeugt, um eine Inaktivierung von Enzymen zu bewirken.

7. Verfahren nach einem der vorangehenden Ansprüche, **dadurch gekennzeichnet, dass** die Flüssigkeit im Wesentlichen gleichförmig in weniger als drei Sekunden auf die Behandlungstemperatur erwärmt wird.

8. Verfahren nach einem der vorangehenden Ansprüche, **dadurch gekennzeichnet, dass** das elektrische Feld durch die gleiche Energiequelle erzeugt wird, die die Flüssigkeit erwärmt.

9. Verfahren nach einem der vorangehenden Ansprüche, **dadurch gekennzeichnet, dass** das elektrische Feld und die Aufwärmung durch eine elektromagnetische Strahlung vom Typ der Mikrowellen erzeugt werden.

10. Verfahren nach einem der Ansprüche 1 bis 8, **dadurch gekennzeichnet, dass** die Erwärmung der Flüssigkeit durch eine Induktionswirkung bei niedriger Frequenz bewirkt wird.

11. Verfahren nach einem der Ansprüche 1 bis 8, **dadurch gekennzeichnet, dass** das elektrische Feld durch ein gleichpoliges elektrisches Feld erzeugt wird.

12. Verfahren nach einem der Ansprüche 1 bis 8, **dadurch gekennzeichnet, dass** das elektrische Feld durch ein elektrisches Gleichfeld erzeugt wird.

13. Verfahren nach einem der vorangehenden Ansprüche, **dadurch gekennzeichnet, dass** das Sterilisationsverfahren auf dicht verschlossene, eine zu sterilisierende Flüssigkeit enthaltende Flaschen angewendet wird.

14. Verfahren nach einem der vorangehenden Ansprüche, **dadurch gekennzeichnet, dass** die Flasche mit einer Geschwindigkeit von 1000 U/min oder mehr um ihre eigene Achse gedreht wird.

15. Verfahren nach einem der vorangehenden Ansprüche, **dadurch gekennzeichnet, dass** der Behälter der Wirkung mehrerer elektrischer Feldimpulse ausgesetzt wird, wobei jeder Impuls einer anderen Position eines im Behälter vorliegenden Gasraumes entspricht.

16. Verfahren nach einem der vorangehenden Ansprüche, **dadurch gekennzeichnet, dass** das Verfahren auf feste Gegenstände angewendet wird, die in eine Flüssigkeit eingetaucht sind oder mit ihr in Berührung stehen.

17. Verfahren nach einem der vorangehenden Ansprüche, **dadurch gekennzeichnet, dass** die thermische Trägheit der zu sterilisierenden Flüssigkeit durch den Zusatz von Elementen aus dielektrischen Materialien modifiziert wird, die eine höhere mittlere Wärmekapazität als die zu sterilisierende Flüssigkeit besitzen.

18. Verfahren nach einem der vorangehenden Ansprüche, **dadurch gekennzeichnet, dass** nach Anwendung des elektrischen Feldes die Flüssigkeit durch einen Wärmetauscher abgekühlt wird, der mit einer Vorerwärmvorrichtung stromauf von der Vorrichtung zur Anwendung des elektrischen Feldes gekoppelt ist.

19. Verfahren nach einem der vorangehenden Ansprüche, **dadurch gekennzeichnet, dass** die Zeitdauer, während der die Flüssigkeit dem elektrischen Feld ausgesetzt wird, weniger als eine Sekunde beträgt.

20. Vorrichtung zur Umsetzung des Verfahrens nach einem der vorangehenden Ansprüche, **dadurch gekennzeichnet, dass** die Vorrichtung ein kinematisches System (4) für die Positionierung und Verschiebung des flüssigen Erzeugnisses (2) sowie eine entlang des kinematischen Systems (4) angeordnete Sterilisationsstation (6) umfasst, wobei die Sterilisationsstation eine elektrische Energiequelle, um in der die Sterilisationsstation durchquerenden Flüssigkeit ein elektrisches Feld von 10² V/cm bis 10⁴ V/cm zu erzeugen, sowie einen Ultraschallgenerator umfasst.

21. Vorrichtung nach dem vorangehenden Anspruch, **dadurch gekennzeichnet, dass** sie eine Aufheizstation (5) stromauf von der Sterilisationsstation umfasst.

22. Vorrichtung nach dem vorangehenden Anspruch, **dadurch gekennzeichnet, dass** sie eine Abkühlstation (7) stromab von der Sterilisationsstation umfasst.

23. Vorrichtung nach dem vorangehenden Anspruch, **dadurch gekennzeichnet, dass** die Aufheizstation und die Abkühlstation mit einem Wärmetauscher ausgerüstet sind, um die Wärme der Abkühlstation zurückzugewinnen und für die Erwärmung der Flüssigkeit in der Aufheizstation zu verwenden.

24. Vorrichtung nach einem der Ansprüche 20 bis 23, **dadurch gekennzeichnet, dass** die elektrische Energiequelle eine Leistung besitzt, die geeignet ist, die zu sterilisierende Flüssigkeit auf mindestens 60 °C zu erwärmen und ein elektrisches Feld von etwa 10³ V/cm in der zu sterilisierenden Flüssigkeit zu erzeugen.

25. Vorrichtung nach einem der Ansprüche 20 bis 23, **dadurch gekennzeichnet, dass** die elektrische Energiequelle ein Induktionselement ist, das ein elektromagnetisches Feld erzeugt.

26. Vorrichtung nach einem der Ansprüche 20 bis 23, **dadurch gekennzeichnet, dass** die elektrische Energiequelle zumindest ein Paar von Elektroden umfasst, die zu beiden Seiten der zu sterilisierenden Flüssigkeit angeordnet und dafür geeignet sind, ein kapazitives Feld zu erzeugen.

27. Vorrichtung nach einem der Ansprüche 20 bis 23, **dadurch gekennzeichnet, dass** die elektrische Energiequelle ein Mikrowellengenerator ist.

28. Vorrichtung nach dem vorangehenden Anspruch, **dadurch gekennzeichnet, dass** die Sterilisationsstation einen Wellenleiter umfasst, der an den Mikrowellengenerator angeschlossen ist, wobei der Wellenleiter einen Kammerabschnitt (25) umfasst, in dem ein oder mehrere gekrümmte Wellenreflektoren (26) angeordnet sind, und wobei die Sterilisationsstation ausserdem einen Kanal (13') umfasst, in dem die zu sterilisierende Flüssigkeit (2) von unten nach oben durch den Kammerabschnitt (25) fliesst.

29. Vorrichtung nach dem vorangehenden Anspruch, **dadurch gekennzeichnet, dass** der Kanal 13' einen schlangenförmigen Abschnitt umfasst, der im Inneren des Kammerabschnitts des Wellenleiters angeordnet ist.

30. Vorrichtung nach einem der Ansprüche 20 bis 27, **dadurch gekennzeichnet, dass** sie stromauf von der Sterilisationsstation einen Mischer umfasst, der es ermöglicht, Zusätze hinzuzugeben, die die mittlere thermische Trägheit der behandelten Flüssigkeit erhöhen bzw. verringern, sowie stromab eine Trennstation, die es ermöglicht, die behandelte Flüssigkeit vom Zusatz abzutrennen.

31. Vorrichtung nach einem der Ansprüche 20 bis 28, **dadurch gekennzeichnet, dass** die Sterilisationsstation einen Wärmetauscher umfasst, der es ermöglicht, die Aufheiztemperatur der behandelten Flüssigkeit zu begrenzen.

32. Vorrichtung nach Anspruch 27, **dadurch gekennzeichnet, dass** die Sterilisationsstation einen Behandlungskanalabschnitt (29) umfasst, in dem sich die mit der zu sterilisierenden Flüssigkeit gefüllten Behälter (3) bewegen, wobei der Behandlungskanalabschnitt über eine mit Schlitzen (37) versehene Wand mit einem Wellenleiter (27) des Mikrowellengenerators (1') verbunden ist.

33. Vorrichtung nach dem vorangehenden Anspruch, **dadurch gekennzeichnet, dass** der Behandlungskanalabschnitt stromauf und stromab mit einer Vorrichtung zur Abschirmung von Mikrowellen versehen ist, die ein mit Schaufeln (32) versehendes Drehkreuz umfasst, das die Mikrowellen abblockt.

34. Vorrichtung nach Anspruch 32 oder 33, **dadurch gekennzeichnet, dass** der Behandlungskanalabschnitt (29) um einen Winkel α geneigt ist, der es Behältern ermöglicht, sich durch Schwerkraft den Kanalabschnitt entlang zu bewegen.
